# EUROPEAN PATENT SPECIFICATION

(11) **EP 2 925 285 B1**
(45) Date of publication and mention of the grant of the patent: **08.08.2018**
(21) Application number: 12794353.8
(22) Date of filing: 03.12.2012
(51) Int. Cl.: A61K 8/362, A61Q 11/00, A61K 8/21

(54) **ORAL CARE COMPOSITION**
MUNDPFLEGEZUSAMMENSETZUNG
COMPOSITION D'HYGIÈNE BUCCALE

(43) Date of publication of application: 07.10.2015
(73) Proprietor: GABA International Holding GmbH, 4106 Therwil (CH)
(72) Inventor: POTH, Tilo, 69469 Weinheim (DE); BRUNELLA, Andre, CH-4143 Dornach (CH); EICHLER, Robert, CH-4053 Basel (CH)
(74) Representative: Wibbelmann, Jobst
(86) International application number: PCT/EP2012/074286
(87) International publication number: WO 2014/086391

(56) References cited:
- EP-A2- 0 162 574
- WO-A1-2004/039343
- US-A1- 2003 124 068

## Description

### Field of the Invention

This invention relates to an oral care composition comprising a fluoride ion source and a buffer. This invention also relates to a method of providing fluoride to the oral cavity of a mammal. The invention further relates to a method of treating or preventing a disease or condition of the oral cavity of a mammal. The invention further relates to the use of a buffer for controlling the pH of an oral care composition comprising fluoride.

### Background of the Invention

It is commonly accepted that fluoride anti caries efficacy is related to the prior formation of a CaF₂ depot (ideally in form of a layer) on the enamel surface (Rølla G., ACTA ODONTOL SCAND 46 (1988)). For efficient formation of CaF₂, conditions need to be slightly acidic since under neutral pH conditions, calcium phosphates form more readily than CaF₂ even with the little phosphate that is available in saliva. Due to the relatively high buffer capacity of saliva (a consequence of the high level of carbonate ions), intra oral pH conditions are rarely kept at the advantageous slightly acidic level for the entire duration of product application.

Fluoride can be provided by oral care products. The majority of oral care products are formulated at neutral pH. Therefore, they do not effectively form CaF₂ layers and there is poor fluoride uptake onto the enamel surface of the teeth. A few oral care products are formulated at a slightly acidic pH, but do not maintain the intra oral pH at the desired level during application of the oral care product.

EP0691124 discloses attempts to increase fluoride uptake by adding polymers to an oral care composition. A copolymer of N-vinylpyrrolidone and acrylic acid is used to improve the bioadhesion of bactericidal compounds and/or to provide an enhanced fluoride uptake. US-A-2003/124068 discloses an oral preparation. WO-A-2004/ 039343 discloses an oral preparation and chewing gum. EP-A-0162574 discloses oral hygiene compositions.

It would be desirable to improve the fluoride uptake from fluoride-containing oral care products. It has now surprisingly been found that fluoride uptake from oral care products can be increased by providing an oral care composition comprising a buffer that can counteract the buffering effect of saliva and hold the pH of the oral care composition at the slightly acidic level necessary to ensure formation of CaF2 layers. The buffer is able to buffer the oral care composition at the desired pH and also yield a stable product.

### Summary of the Invention

The present invention concerns in oral care composition comprising an orally acceptable vehicle, an amine fluoride and a buffer having a pKₐ of less than 7.0 wherein the buffer comprises an aqueous solution of an acid and a salt of the acid, and wherein the ratio of acid:salt is between 2:1 and 1:2,wherein the pH of the oral composition is greater than 3.5 and less than 5.0, and wherein the oral composition has an acid number greater than 6.0, and wherein the acid is selected from succinic acid, tartaric acid, malic acid, lactic acid, fumaric acid, glutamic acid, suberic acid, adipic acid, sebacic acid, glutaric acid, azelaic acid and combinations thereof, and wherein the oral care composition is a dentifrice, toothpaste, mouthrinse, mouthwash, strip or a solid or liquid gel. Further embodiments of the invention are set forth in the attached claims.

Optionally, the buffer is present at a level from 0.05 to 5.00 weight % or 0.1 to 3.00 weight % based on the total weight of the composition. Further optionally, the buffer is present at a level from 0.50 to 2.00 % weight % based on the total weight of the composition. In one embodiment, the buffer is present at a level of 0.7 to 1.8
weight %. In another embodiment, the buffer is present at a level of 1.4 to 1.6 weight %.

Optionally, the fluoride ion is selected from sodium fluoride, potassium fluoride, stannous fluoride, amine fluoride and combinations thereof. Optionally the fluoride ion is present at a level from 100 ppm to 10000 ppm. Further optionally, the fluoride ion is present a level from 150 ppm to 2000 ppm, 150 ppm to 1800 ppm or 150 to 1500 ppm. Further optionally, the fluoride ion is present at a level of 250 ppm to 2000 ppm, 250 ppm to 1800 ppm or 250 ppm to 1500 ppm.

Optionally, the pH of the oral composition is from 4.2 to 4.8, from 4.3 to 4.7, from 4.4 to 4.7 or from 4.4 to 4.6. Optionally the pH of the composition is 4.5.

Optionally, the buffer has a pKₐ from 2.5 to 6.0. Further optionally the buffer has a pKₐ from 3.5 to 5.6, from 4.0 to 5.6 or from 4.2 to 5.6.

Optionally the acid number is greater than or equal to 6.45, 7.0, 8.0, 9.0 or 10.0. Optionally the acid number is less than 25 or further optionally less than 18.

The oral composition is a dentifrice, toothpaste, mouthrinse, mouthwash, strip or a solid or liquid gel. The oral composition preferably is a dentifrice or mouthrinse.

Optionally the buffer is selected from an aqueous solution of succinic acid and the sodium or potassium salt of succinic acid, an aqueous solution of tartaric acid and the sodium or potassium salt of tartaric acid, an aqueous solution of malic acid and the sodium or potassium salt of malic acid, an aqueous solution of fumaric acid and the sodium or potassium salt of fumaric acid, an aqueous solution of glutamic acid and the sodium or potassium salt of glutamic acid, an aqueous solution of suberic acid and the sodium or potassium salt of suberic acid, an aqueous solution of adipic acid and the sodium or potassium salt of adipic acid, an aqueous solution of sebacic acid and the sodium or potassium salt of sebacic acid, an aqueous solution of glutaric acid and the sodium or potassium salt of glutaric acid and an aqueous solution of azelaic acid and the sodium or potassium salt of azelaic acid and combinations thereof; the buffer is present at a level from 0.05 to 5.00 weight % based on the total weight of the composition; the pH of the composition is from 4.2 to 4.8 and the oral care composition has an acid number greater than 6.0.

Also disclosed herein is a method of providing fluoride ions to the oral cavity of a mammal comprising contacting a composition according to the invention with the oral cavity.

Also disclosed herein is a method of treating or preventing a disease or condition of the oral cavity of a mammal comprising applying a composition according to the invention to the oral cavity.

Also disclosed herein is a method of decreasing dental cavity formation in mammalian teeth comprising applying a composition according to the invention to the oral cavity of a mammal.

Also disclosed herein is a method of re-mineralizing teeth in a mammal comprising applying a composition according to the invention to the oral cavity of a mammal.

According to a further aspect of the invention there is provided a composition according to the invention for use in a method of providing fluoride ions to the oral cavity of a mammal.

According to a further aspect of the invention there is provided a composition according to the invention for use in a method of treating or preventing a disease or condition of the oral cavity of a mammal,

According to a further aspect of the invention there is provided a composition according to the invention for use in a method of decreasing dental cavity formation in mammalian teeth.

According to a further aspect of the invention there is provided a composition according to the invention for use in a method of re-mineralizing mammalian teeth.

According to a further aspect of the invention there is provided use of a buffer having a pKₐ of less than 7.0 for controlling the pH of an oral care composition comprising a fluoride ion source, the buffer controlling the pH of the composition such that the pH is greater than 3.5 and less than 5.0 and buffering the composition such that the oral care composition has an acid number greater than or equal to 4.5.

The compositions may contain additional therapeutic and non-therapeutic components.

This invention is predicated on the finding by the present inventors that in oral care compositions comprising fluoride, fluoride uptake from the oral care composition can be increased by including a buffer in the oral care composition to maintain the intra oral pH at a slightly acidic level during application of the oral care composition.

### Detailed Description of the Invention

The features and benefits of the invention are illustrated by reference to the preferred embodiments. Accordingly, the invention expressly should not be limited to such preferred embodiments illustrating some possible non-limiting combination of features that may exist alone or in other combinations of features. It should be understood that the detailed description and specific examples, while indicating embodiments of the invention, are intended for purposes of illustration only and are not intended to limit the scope of the invention.

As referred to herein, all compositional percentages are by weight of the total composition, unless otherwise specified.

### Orally acceptable vehicle

The dentifrice composition according to the invention comprises an orally acceptable vehicle. As used herein, an "orally acceptable vehicle" refers to a material or combination of materials that are safe for use in the compositions of the invention, commensurate with a reasonable benefit/ risk ratio.

The composition may contain any conventional excipients or carriers, although these will vary depending on the dosage form or means of dosage selected. Excipients or carriers can include, for example, humectants, glycerin, sorbitol, xylitol, and/or propylene glycol, water or other solvents.

Surfactants may be included, if desired. Examples of suitable surfactants include water-soluble salts of higher fatty acid monoglyceride monosulfates, such as the sodium salt of monosulfated monoglyceride of hydrogenated coconut oil fatty acids; higher alkyl sulfates such as sodium lauryl sulfate; alkyl aryl sulfonates such as sodium dodecyl benzene sulfonate; higher alkyl sulfoacetates, such as sodium lauryl sulfoacetate; higher fatty acid esters of 1,2-dihydroxypropane sulfonate; and the substantially saturated higher aliphatic acyl amides of lower aliphatic amino carboxylic compounds, such as those having 12-16 carbons in the fatty acid, alkyl or acyl radicals; and the like. Examples of the last mentioned amides include N-lauryl sarcosine, and the sodium, potassium and ethanolamine salts of N-lauryl, N-myristoyl, or N-palmitoyl sarcosine. Others include, for example, nonanionic polyoxyethylene surfactants, such as Polyoxamer 407, Steareth 30, Polysorbate 20, and castor oil; and amphoteric surfactants, such as cocamidopropyl betaine (tegobaine), and cocamidopropyl betaine lauryl glucoside; condensation products of ethylene oxide with various hydrogen containing compounds that are reactive therewith and have long hydrocarbon chains (e.g., aliphatic chains of from 12 to 20 carbon atoms), which condensation products (ethoxamers) contain hydrophilic polyoxyethylene moieties, such as condensation products of poly (ethylene oxide) with fatty acids, fatty alcohols, fatty amides and other fatty moieties, and with propylene oxide and polypropylene oxides.

In an embodiment, the oral composition includes a surfactant system that is sodium lauryl sulfate (SLS).
Optionally, the orally acceptable vehicle comprises sorbitol which is present in an amount of from 10 to 25 wt% based on the weight of the composition, further optionally from 12 to 18 wt% based on the weight of the composition.

### Fluoride Ion Source

Any orally acceptable particulated fluoride ion can be used, including potassium, sodium and ammonium fluorides and monofluorophosphates, stannous fluoride, indium fluoride, amine fluorides such as olaflur (N'-octadecyltrimethylendiamine-N,N,N'-tris(2-ethanol)-dihydrofluoride), and mixtures thereof. One or more fluoride ions are optionally present in an amount providing a clinically efficacious amount of soluble fluoride ion to the oral composition. Optionally the fluoride ion is present at a level from 100 ppm to 10000 ppm based on the total weight of the composition. Further optionally, the fluoride ion is present a level from 150 ppm to 2000 ppm, 150 ppm to 1800 ppm or 150 to 1500 ppm. Further optionally, the fluoride ion is present at a level of 250 ppm to 2000 ppm, 250 ppm to 1800 ppm or 250 ppm to 1500 ppm.

Optionally, the toothpaste composition further comprises sodium monofluorophosphate in an amount of from 0.75 to 1.5 wt% based on the weight of the composition.

### Buffer

A buffer in an aqueous solution of a weak acid and its conjugate base or a weak base and its conjugate acid. Buffer solutions maintain the pH of a solution by means of the equilibrium between the acid and its conjugate base (or the base and its conjugate acid).

The pKₐ value of an acid is a measure of the strength of an acid in solution. The Kₐ or acid dissociation constant (also known as the acidity constant or acid-ionization constant) is the equilibrium constant for the dissociation of an acid (HA) into its conjugate base (A-) and a proton (H⁺). The pKₐ is the logarithmic measure and is equal to -log₁₀Kₐ. Polyprotic acids that can lose more than proton can have successive pKₐ dissociation values for dissociation of successive protons. The pKₐ of an acid can be determined by titration. The pKₐ is determined at 25°C unless otherwise stated. Apart from the required acid-base properties the buffer needs to be sufficiently soluble in the oral care formula backbone. Acids that are rather poorly soluble as a free acid but readily soluble in the form of an alkali salt form a reservoir of acid that is not in solution and is thus behaving neutral on taste. It is, however, available for buffering. While buffering takes place during the intra oral application of the product the acid solubilizes at the same rate as the protons are consumed by saliva. Since the concentration of free solubilized acid remains small during that process the influence on taste is minimal. Fumaric acid is an acid that works according to the mechanism described.

### Acid Number

The acid number is a measure quantifying the buffering capacity of a product. The acid number or acid value expresses in milligrams the quantity of potassium hydroxide needed to neutralise the free acids present in 1 g of the product. The determination can be carried out e.g. by an adaptation of the method according to the protocol described in the European Pharmacopoeia 7.0 section 2.5.1. dissolving samples in water instead of ethanol/ petroleum ether; and monitoring the end point with a pH electrode instead of with phenolphthalein indicator.

Sample preparation: Weigh sample in a 150ml beaker. The amount of sample depends on the expected acid value. As a guideline use approximately 5.0g for toothpaste or gels or approximately 10mL to 20mL for mouth rinses. Add 50mL of water pH 9 prepared previously and stir the sample until it's completely dissolved or until the resulting suspension is homogeneous. Titrate slowly and under stirring the sample solution using a 0.1N potassium hydroxide solution. End point of the titration is reached, when the pH of the solution is exactly 9.00 and it remains stable for at least 15 seconds (the drift should be less than 0.1 pH units/minute, or less than 0.025 pH units during 15 seconds). Calculation of the acid value is done as described above. This parameter yields the amount of protons neutralized but does not provide information on the pH at which buffering occurs. A product can have a high acid number but would do little to keep the pH constant at a particular pH of interest. Therefore the buffer substance must additionally have a pKₐ close to the desired pH that is to be kept constant. Ideally, the pKₐ of the buffer is within the range of the desired pH ± 1. The acid number of the oral composition is at least greater than or equal to 4.5. Optionally, the acid number is greater than or equal to 6.0. Optionally the acid number is greater than or equal to 6.45, 7.0, 8.0, 9.0 or 10.0. Optionally the acid number is less than 25 or further optionally less than 18.

### pH

In order to increase the fluoride uptake from the composition, the compositions of the present invention are buffered to maintain the pH at a slightly acidic level. By "slightly acidic" it is meant that the oral composition has a pH of less than 7.0 but greater than 3.5. An example of a pH that is considered slightly acidic is a pH of from 3.5 to 6.0 or from 4.0 to 6.0, or from 3.5 to 5.0 or from 4.0 to 5.0. A pH of 4.5 is considered to be slightly acidic in the context of this invention. Measurement of the pH can be carried by a procedure based on the method described in the European Pharmacopoeia 7.0, section 2.2.36. The fluoride concentration is measured at a constant ionic strength by using a fluoride selective electrode. Constant ionic strength in the measuring solution is ensured by the use of a TISAB (total ionic strength adjustment buffer). Fluoride is always measured as ionic fluoride (F-). Analysis is performed by measuring the difference in electrical potential of fluoride selective (ISE) and reference electrode immersed in the measuring solution.

Sample preparation for toothpaste: Weigh a sample of approximately 200 mg to the nearest 0.1 milligram on the bottom of a 50mL plastic beaker and add exactly 20.0mL deionized water. Let stir for 5 minutes or until the sample is completely dissolved and the resulting suspension is homogeneous. Proceed to measurement of the pH as described above.

Sample preparation for mouthrinse: Weigh a sample of approximately 1000 mg to the nearest milligram in a 50mL plastic beaker and add 19.0mL deionized water. Stir until the sample solution is homogenous. Proceed to measurement of the pH as described above.

Optionally the pH of the oral composition is greater than 4.0. Further optionally, the pH of the oral composition is from 4.2 to 4.8. Further optionally, the pH of the oral composition is 4.5.

### Thickening system

The compositions of the invention may optionally comprise an additional orally acceptable thickening agent, selected from one or more of, without limitation, silica, polyvinyl pyrrolidone, which may be linear or cross-linked, carbomers, also known as carboxyvinyl polymers, carrageenans, also known as Irish moss and more particularly -carrageenan (iota-carrageenan), high molecular weight polyethylene glycols (such as CARBOWAX®, available from The Dow Chemical Company), cellulosic polymers such as hydroxyethylcellulose, carboxymethylcellulose (CMC) and salts thereof, e.g., CMC sodium, natural gums such as karaya, xanthan gum, gum arabic and tragacanth, and colloidal magnesium aluminum silicate and mixtures of the same.

The cellulose polymer may be selected from one or more of hydroxypropylmethyl cellulose (HPMC), hydroxyethylpropyl cellulose (HEPC), hydroxybutylmethyl cellulose (HBMC), and carboxymethyl cellulose (CMC).

Optionally, such additional thickening agents are present in a total amount of 0.1 wt% to 50 wt%, for example 0.1 wt% to 35 wt% or 1 wt% to 15 wt%, based on the weight of the composition.

The compositions of the invention may also include a polymeric adherent material that attaches to the surface of a mammalian tooth and/or to the heterogeneous biofilm which also may be present on a tooth's surface. Attachment may occur by any means, such as ionic interaction, van der Waals forces, hydrophobic-hydrophilic interactions, etc. The adherent material may be, for example, any homopolymers or copolymers (hereinafter referred to collectively as a "polymers") that adhere to the surface of a tooth. For example, the composition may additionally include poly (ethylene oxide) polymers (such as POLYOX from Dow Chemical), linear PVP and cross-linked PVP, PEG/PPG copolymers (such as BASF Pluracare L1220), ethylene oxide (EO) - propylene oxide (PO) block copolymers (such as polymers sold under the trade mark Pluronic available from BASF Corporation), ester gum, shellac, pressure sensitive silicone adhesives (such as BioPSA from Dow-Corning), methacrylates, or mixtures thereof. In an embodiment, a copolymer comprises (PVM/MA). In an embodiment, a copolymer comprises poly (methylvinylether/maleic anhydride). In another embodiment, a copolymer comprises poly (methylvinylether/maleic acid). In another embodiment, a copolymer comprises poly (methylvinylether/maleic acid) half esters. In another embodiment, a copolymer comprises poly (methylvinylether/maleic acid) mixed salts.

In some embodiments, the composition further comprises polymer thickeners selected from (i) polyethylene glycol, (ii) polyethylene glycol - polypropylene glycol block co-polymers having a molecular weight of at least 5000, and (iii) combinations thereof. In some embodiments, the composition comprises an ethylene oxide, propylene oxide block co-polymer of formula (ethylene oxide)ₓ-(propylene oxide)_{y} wherein x is an integer of 80-150, e.g. 100-130, e.g. 118, and y is an integer 30-80, e.g. 60-70, e.g. 66, having an average molecular weight of greater than 5000, e.g., 8000 -13000 Da, e.g. 9800;
In some embodiments, the composition comprises an ethylene oxide, propylene oxide block co-polymer of average molecular weight greater than 5000 Da, being substantially free of an ethylene oxide, propylene oxide block co-polymer of average molecular weight less than 5000 Da. Optionally, the ethylene oxide, propylene oxide block co-polymer is present in an amount of from 5 wt % to 10 wt % based on the weight of the composition. Block copolymers of ethylene oxide / propylene oxide are useful, but higher molecular weight, e.g., > 5000Da are preferred, e.g. including PLURACARE® L1220 (available from BASF, Wyandotte, Mich., United States of America). In some embodiments, the composition further comprises polyethylene glycol of average molecular weight 400 to 800 Da, e.g., 600 Da. Low or medium molecular weight polyethylene glycol, e.g., PEG 400, PEG 600, PEG 800, PEG 1000 and mixtures thereof are useful in the compositions of some embodiments of the invention. Further optionally, the polyethylene glycol may be present in an amount of from 5 wt % to 15 wt % based on the weight of the composition.

Polymers of any molecular weight may be used, including, for example molecular weights of 50,000 to 500,000, 500,000 to 2,500,000 or 2,500,000 to 10,000,000 (calculated by either number average or weight average).
Commercially-available polymers may be used in the present invention. It is understood that over time, the exact size, weight and/or composition of a commercially-available polymer may change. Based on the disclosure set forth herein, the skilled artisan will understand how to determine whether such polymers are useful in the invention.

### Abrasive system

Some embodiments may further comprise an abrasive. Optionally, the oral care compositions may comprise from 5 to 15 wt% abrasive based on the weight of the composition. Where abrasives are present, the average particle size is generally 0.1 to 30 microns, for example 1 to 20 or 5 to 15 microns.

The abrasive may comprise a calcium abrasive, such as a calcium phosphate salt, (e.g., calcium pyrophosphate, dicalcium orthophosphate dihydrate, tricalcium phosphate, calcium pyrophosphate), calcium sulfate and/or calcium polymetaphosphate. The oral composition may comprise abrasive particulates selected from sodium bicarbonate, silica, iron oxide, aluminium oxide, perlite, plastic particles, e.g., polyethylene, and combinations thereof. Any type of silica may be used, such as hydrated silica, precipitated silica or silica gel. Optionally, the composition may comprise, as a thickener and also as an abrasive, silica particles in an amount of from 1 to 3 wt% based on the weight of the composition. In a typical embodiment, the calcium abrasive comprises calcium pyrophosphate. In another embodiment, the calcium abrasive comprises calcium carbonate. Optionally, the composition is a toothpaste comprising a calcium pyrophosphate abrasive. Further optionally, the calcium pyrophosphate is present in an amount of from 10 wt % to 20 wt % based on the weight of the composition.

The compositions may comprise silica that has a particle size and an amount and distribution in the composition so that the silica has a dual function, and functions not only as a dentin tubule-occluding particulate but also as an abrasive particulate. Such a dual function particulate may be provided by a commercially available silica such as INEOS AC43, available in commerce from Ineos Silicas, Warrington, United Kingdom. In an embodiment, such silica has a median particle size less than 8 µm, for example from 3 µm to 5 µm.

The compositions of the present invention may further comprise an abrasive useful for example as a polishing agent. Any orally acceptable abrasive can be used, but type, fineness, (particle size) and amount of abrasive should be selected so that tooth enamel is not excessively abraded in normal use of the composition. Suitable abrasives include silica, for example in the form of precipitated silica or as admixed with alumina, insoluble phosphates, and mixtures thereof. Among insoluble phosphates useful as abrasives are orthophosphates, polymetaphosphates and pyrophosphates. Illustrative examples are dicalcium orthophosphate dihydrate, calcium pyrophosphate, calcium pyrophosphate, tricalcium phosphate, calcium polymetaphosphate and insoluble sodium polymetaphosphate.
In an embodiment, the abrasive particles may be initially present in the toothpaste composition having the desired particle size, or may be initially present in the composition at a larger size, so long as the structure of the particles is such that it fractures or breaks into the desired particle size upon application of mechanical force by, e.g., a toothbrush, when brushing.

### Additional ingredients

Flavorants, sweeteners, colorants, foam modulators, mouth-feel agents and others additively may be included if desired, in the composition. These may be included in any combination of one or more additional ingredients.

The compositions of the present invention may comprise a surface active agent (surfactant). Suitable surfactants include without limitation water-soluble salts of C₈₋₂₀ alkyl sulfates, sulfonated monoglycerides of C₈₋₂₀ fatty acids, sarcosinates, taurates, sodium lauryl sulfate, sodium cocoyl monoglyceride sulfonate, sodium lauryl sarcosinate, sodium lauryl isoethionate, sodium laureth carboxylate and sodium dodecyl benzenesulfonate, and cocoamidopropyl betaine. In some embodiments, the composition may additionally comprise a surfactant, e.g., sodium lauryl sulfate (SLS).

The oral care composition may include any other therapeutic, cosmetic, and/or aesthetic materials as may be desired. Examples include desensitizing agents (e.g. a nitrate salt, an arginine ester, a bicarbonate salt, potassium nitrate, an arginine-bicarbonate-phytate complex, potassium citrate, and arginine, among others), a chemical whitening agent (such as a peroxide releasing compound), an opaque whitening agent (such as hydroxyapatite) and an anticalculus agent.

The composition according to the invention may also comprise one or more further agents typically selected from an anti-plaque agent, an anti-caries agent, a whitening agent, desensitizing agent, antimicrobial agent, antibacterial agent, cleaning agent, a flavouring agent, a sweetening agent, adhesion agents, viscosity modifiers, diluents, surfactants, foam modulators, emulsifiers, abrasives, pH modifying agents, humectants, mouth feel agents, colorants, preservatives, tartar control (anticalculus) agent, saliva stimulating agent, nutrient and combinations thereof.

The humectants may be selected from for example glycerin, propylene glycol or a combination thereof. In some embodiments, the oral care composition comprises from 20 to 60 wt% humectant based on the weight of the composition. In some embodiments, the composition comprises propylene glycol in an amount of from 10wt % to 20 wt % based on the weight of the composition. In some embodiments, the composition comprises glycerin in an amount of from 25wt % to 40 wt % based on the weight of the composition.

Suitable anticalculus agents include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. Typically, the anticalculus agent is present at 0.1 wt % to 30 wt% based on the weight of the composition. The oral composition may include a mixture of different anticalculus agents. In some embodiments, the composition additionally comprises a tartar control agent, e.g., selected from tetrasodium pyrophosphate (TSPP) and sodium tripolyphosphate (STPP). In one embodiment, tetrasodium pyrophosphate (TSPP) and sodium tripolyphosphate (STPP) are used. The anticalculus agent comprises TSPP at 1-2 wt % and STPP at 7 wt % to 10 wt %, each based on the weight of the composition.

The compositions may include a stannous ion or a stannous ion source. Suitable stannous ion sources include without limitation stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide and the like. One or more stannous ion sources are optionally and illustratively present in a total amount of 0.01% to 10 wt %, for example 0.1 wt % to 7 wt % or 1 wt % to 5 wt %, each based on the weight of the composition.

In some embodiments, the compositions of the invention optionally comprise an antimicrobial (e.g., antibacterial) agent, e.g., triclosan. A further illustrative list of useful antibacterial agents is provided in such as those listed in U.S. Pat. No. 5,776,435 to Gaffar et al. One or more antimicrobial agents are optionally present in an antimicrobial effective total amount, typically 0.05 wt % to 10 wt %, for example 0.1 wt % to 3 wt %, each based on the weight of the composition.

In some embodiments, the compositions of the invention optionally comprise an antioxidant. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

The compositions of the invention may optionally comprise a sialagogue or saliva-stimulating agent, an antiplaque agent, an anti-inflammatory agent, and/or a desensitizing agent.

It is preferred that the when the oral care composition is a dentifrice, the vehicle ingredients in particular provide a dentifrice with a viscosity of 10,000 CPS to 700,000 CPS, preferably 30,000 CPS to 300,000 CPS.

Colorants may be used in a single phase toothpaste or a two-phase toothpaste for forming a striped toothpaste. Such colorants may be selected from pigments, dyes, lakes and agents imparting a particular luster or reflectivity such as pearling agents. In various embodiments, colorants are operable to provide a white or light-colored coating on a dental surface, to act as an indicator of locations on a dental surface that have been effectively contacted by the composition, and/or to modify appearance, in particular color and/or opacity, of the composition to enhance attractiveness to the consumer. Any orally acceptable colorant can be used, including FD&C dyes and pigments, talc, mica, magnesium carbonate, magnesium silicate, silica, titanium dioxide, zinc oxide, red, yellow, brown and black iron oxides, ferric ammonium ferrocyanide, manganese violet, ultramarine, titanated mica, bismuth oxychloride, and mixtures thereof. One or more colorants are optionally present in a total amount of 0.001 % to 20%, for example 0.01% to 10% or 0.1% to 5%.

Optionally, the toothpaste composition further comprises titanium dioxide in an amount of from 0.05 to 0.15 wt% based on the weight of the composition. Such titanium dioxide addition has been found to whiten the slightly yellowish appearance of the toothpaste caused by the addition of the guar gum binder.

While ingredients are sometimes identified herein by category, e.g., humectant, antioxidant, thickener, etc., this identification is for convenience and clarity, but is not intended to be limiting. All of the ingredients in the compositions may have functions in addition to their primary function, and may contribute to the overall properties of the composition, including its stability, efficacy, consistency, mouthfeel, taste, odor and so forth.

It is understood that while general attributes of each of the above categories of materials may differ, there may be some common attributes and any given material may serve multiple purposes within two or more of such categories of materials. Preferably, the carrier is selected for compatibility with other ingredients of the composition.

### Preparation

The toothpaste composition of the invention may be prepared by any means known in the art.

### Examples

Toothpaste formulation based on a silica base were prepared by using various concentrations of 1:1 buffer solutions prepared from the acids and their sodium/potassium salts. The acid/salt mixtures have been employed at levels from 0.5 % to 2.00 % total buffer concentration in the final formulation. The yielding toothpaste met all the stability criteria after accelerated aging from 6 months at 40 °C.

The pastes have been submitted to Enamel Fluoride Uptake (EFU) testing according to FDA Method #40. This method determines the fluoride uptake into caries like subsurface lesions affected by an in vitro treatment of tooth specimen with slurries of toothpastes. The buffered recipes were referenced against toothpastes of identical formula but without the buffering substance. Formulations tested are in Table 1. Results are summarized in Table 2.

**Table 1: Formulations**

| **Ingredient** | **Amount (%)** | | | | | |
|---|---|---|---|---|---|---|
| | 1 | 2 | 3 | 4 | 5 | 6 |
| Amine Fluoride (1.4% F) | 10.0 | 10.0 | 10.0 | 10.0 | 10.0 | 10.0 |
| Succinic Acid | 0.0 | 0.0 | 1.4 | 0.0 | 0.0 | 0.0 |
| Malic Acid | 1.5 | 0.0 | 0.0 | 0.0 | 0.5 | 0.0 |
| Sorbitol 70% | 27.0 | 27.0 | 27.0 | 27.0 | 27.0 | 27.0 |
| Luwax PE 10 M | 11.0 | 11.0 | 11.0 | 11.0 | 11.0 | 11.0 |
| Tixosil | 6.0 | 6.0 | 6.0 | 6.0 | 6.0 | 6.0 |
| Titanium Dioxide | 0.9 | 0.9 | 0.9 | 0.9 | 0.9 | 0.9 |
| Tylose | 2.2 | 2.2 | 2.2 | 2.2 | 2.2 | 2.2 |
| Aerosil R-972 | 1.8 | 1.8 | 1.8 | 1.8 | 0.0 | 1.8 |
| Sylodent TM614 | 0.0 | 0.0 | 0.0 | 0.0 | 1.8 | 0.0 |

**Table 2: EFU data for buffered and non-buffered toothpastes**

| Toothpastes | **pH** | **Buffer** | **Acid Number** | **Level of buffer (%)** | **Enamel Fluoride Uptake (ppm)** |
|---|---|---|---|---|---|
| 1 | 4.5 | Malic acid | 8.44 | 1.53 | 2013±219 |
| 2 | 4.5 | No buffer | 4.30 | 0.00 | 1382±252 |
| 3 | 4.5 | Succinic acid | 10.95 | 1.53 | 2153±193 |
| 4 | 4.5 | No buffer | 4.30 | 0.00 | 1429±183 |
| 5 | 4.5 | Malic acid | 6.45 | 0.50 | 1682±230 |
| 6 | 4.5 | No buffer | 4.30 | 0.00 | 1364±193 |

For all paste with additional buffer the fluoride uptake was significantly higher than for the respective controls. Also a dose effect for the amount of buffer used was observed.

Additionally, selected pastes were tested for the remineralization potential. To do so, specimen derived from human teeth were initially demineralized (baseline) and then alternately exposed to an acid challenge (demineralization pH 5.00, 4 h daily) and a slurry of the toothpaste (remineralization, 4 treatments per day) for 20 days. Between acid exposure and treatments the specimens were stored in saliva. The degree of mineralization of the specimen was investigated by micro hardness measurements (VMH) and compared to the baseline. The higher the gain in hardness the more efficient the remineralization potential of the used product is. Again the pastes were referenced against paste of identical formula but without the buffering substance. Results are summarized in Table 3.

**Table 3: Values for hardness improvements for both buffered and non-buffered toothpastes**

| **Toothpastes** | **pH** | **Buffer** | **Level of buffer (%)** | **Hardness improvement in Vickers hardness numbers at day 20 from baseline** |
|---|---|---|---|---|
| 1 | 4.5 | Malic acid | 1.53 | +30.3±17.8 |
| 2 | 4.5 | No buffer | 0.00 | +16.1±9.8 |
| 3 | 4.5 | Succinic acid | 1.53 | +23.6±6.6 |
| 4 | 4.5 | No buffer | 0.00 | +19.0±5.4 |

In all cases the hardness improvement was significantly higher than for the respective control toothpaste. The results are in accordance with the EFU data. The buffered toothpastes also show an increased remineralization potential compared to state of the art toothpastes.

It can be seen that pastes with increased buffer capacity lead to an improved fluoride uptake in caries-like lesions. Thus pastes according to the invention will have an increased anti-cavity effect compared to state of the art pastes.

## Claims

1. An oral care composition comprising
an orally acceptable vehicle,
an amine fluoride and
a buffer having a pKₐ of less than 7.0
wherein the buffer comprises an aqueous solution of an acid and a salt of the acid, and wherein the ratio of acid:salt is between 2:1 and 1:2,
wherein the pH of the oral composition is greater than 3.5 and less than 5.0, and wherein the oral composition has an acid number greater than 6.0,
and
wherein the acid is selected from succinic acid, tartaric acid, malic acid, lactic acid, fumaric acid, glutamic acid, suberic acid, adipic acid, sebacic acid, glutaric acid, azelaic acid and combinations thereof, and
wherein the oral care composition is a dentifrice, toothpaste, mouthrinse, mouthwash, strip or a solid or liquid gel.

2. The oral care composition according to claim 1 wherein the acid is selected from fumaric acid, succinic acid, malic acid and combinations thereof.

3. The oral care composition according to any preceding claim wherein the ratio of acid:salt is between 1.5:1 and 1:1.5.

4. The oral care composition according to any preceding claim wherein the buffer is present at a level of 0.05 - 5.00 weight % based on the total weight of the composition, optionally at a level of 0.50 - 2.00 % weight % based on the total weight of the composition.

5. The oral care composition according to any preceding claim wherein the amine fluoride is olaflur (N'-octadecyltrimethylendiamine-N,N,N'-tris(2-ethanol)-dihydrofluoride).

6. The oral care composition according to any preceding claim wherein the fluoride ion is present at a level of 100 ppm to 10000 ppm.

7. The oral care composition according to any preceding claim wherein the pH of the oral composition is greater than 4.0, optionally wherein the pH of the oral composition is from 4.2 to 4.8.

8. The oral care composition according to any preceding claim wherein the buffer has a pKₐ from 2.5 to 6.0, optionally from 3.5 to 5.6, further optionally from 4.0 to 5.6, still further optionally between 4.2 and 5.6.

9. The oral care composition according to any preceding claim wherein the acid number is less than 25.

10. The oral care composition according to any preceding claim, wherein the oral composition is a dentifrice or mouthrinse.

11. The oral care composition according to any preceding claim wherein the buffer is selected from an aqueous solution of succinic acid and the sodium or potassium salt of succinic acid, an aqueous solution of tartaric acid and the sodium or potassium salt of tartaric acid, an aqueous solution of malic acid and the sodium or potassium salt of malic acid, an aqueous solution of fumaric acid and the sodium or potassium salt of fumaric acid, an aqueous solution of glutamic acid and the sodium or potassium salt of glutamic acid, an aqueous solution of suberic acid and the sodium or potassium salt of suberic acid, an aqueous solution of adipic acid and the sodium or potassium salt of adipic acid, an aqueous solution of sebacic acid and the sodium or potassium salt of sebacic acid, an aqueous solution of glutaric acid and the sodium or potassium salt of glutaric acid, and an aqueous solution of azelaic acid and the sodium or potassium salt of azelaic acid, and combinations thereof,
wherein the buffer is present at a level from 0.05 to 5.00 weight % based on the total weight of the composition and
wherein the pH of the composition is from 4.2 to 4.8.

12. A composition according to any of claims 1 to 11 for use in a method of providing fluoride ions to the oral cavity of mammal, or for use in a method of treating or preventing a disease or condition of the oral cavity of a mammal, or for use in a method of decreasing dental cavity formation in mammalian teeth, or for use in a method of re-mineralizing mammalian teeth.

13. Use of a buffer having a pKₐ of less than 7.0 wherein the buffer comprises an aqueous solution of an acid and a salt of the acid, and wherein the ratio of acid:salt is between 2:1 and 1:2, for controlling the pH of an oral care composition comprising amine fluoride, the buffer controlling the pH of the composition such that the pH is greater than 3.5 and less than 5.0 and buffering the composition such that the composition has an acid number greater than 6.0, wherein the acid is selected from succinic acid, lactic acid, fumaric acid, glutamic acid, suberic acid, adipic acid, sebacic acid, glutaric acid, azelaic acid and combinations thereof, and wherein the oral care composition is a dentifrice, toothpaste, mouthrinse, mouthwash, strip or a solid or liquid gel.

## Patentansprüche

1. Mundpflegezusammensetzung, umfassend
einen oral annehmbaren Träger,
ein Aminfluorid und
einen Puffer mit einem pKₐ von weniger als 7,0,
worin der Puffer eine wässrige Lösung einer Säure und eines Salzes der Säure umfasst, und wobei das Verhältnis von Säure:Salz zwischen 2:1 und 1:2 ist,
worin der pH-Wert der oralen Zusammensetzung größer als 3,5 und geringer als 5,0 ist, und worin die orale Zusammensetzung eine Säurezahl größer als 6,0 hat,
und
wobei die Säure ausgewählt ist aus Bernsteinsäure, Weinsäure, Äpfelsäure, Milchsäure, Fumarsäure, Glutaminsäure, Korksäure, Adipinsäure, Sebacinsäure, Glutarsäure, Azelainsäure und Kombinationen davon, und
wobei die Mundpflegezusammensetzung eine Zahnpflegezusammensetzung, Zahnpasta, Mundspülung, Mundwäsche, Streifen oder ein festes oder flüssiges Gel ist.

2. Mundpflegezusammensetzung gemäß Anspruch 1, worin die Säure ausgewählt ist aus Fumarsäure, Bernsteinsäure, Äpfelsäure und Kombinationen davon.

3. Mundpflegezusammensetzung gemäß irgendeinem vorhergehenden Anspruch, worin das Verhältnis von Säure:Salz zwischen 1,5:1 und 1:1,5 ist.

4. Mundpflegezusammensetzung gemäß irgendeinem vorhergehenden Anspruch, worin der Puffer bei einer Konzentration von 0,05-5,00 Gew.-%, bezogen auf das Gesamtgewicht der Zusammensetzung, vorliegt, gegebenenfalls bei einer Konzentration von 0,50-2,00 Gew.-%, bezogen auf das Gesamtgewicht der Zusammensetzung.

5. Mundpflegezusammensetzung gemäß irgendeinem vorhergehenden Anspruch, worin das Aminfluorid Olaflur (N'-Octadecyltrimethylendiamin-N,N,N,'-tris(2-Ethanol)-Dihydrofluorid) ist.

6. Mundpflegezusammensetzung gemäß irgendeinem vorhergehenden Anspruch, worin das Fluoridion bei einer Konzentration von 100 ppm bis 10 000 ppm vorliegt.

7. Mundpflegezusammensetzung gemäß irgendeinem vorhergehenden Anspruch, worin der pH-Wert der Mundpflegezusammensetzung größer als 4,0 ist, gegebenenfalls worin der pH-Wert der oralen Zusammensetzung von 4,2 bis 4,8 ist.

8. Mundpflegezusammensetzung gemäß irgendeinem vorhergehenden Anspruch, wobei der Puffer einen pKₐ von 2,5 bis 6,0 hat, gegebenenfalls von 3,5 bis 5,6, weiterhin gegebenenfalls von 4,0 bis 5,6, und weiterhin gegebenenfalls zwischen 4,2 und 5,6.

9. Mundpflegezusammensetzung gemäß irgendeinem vorhergehenden Anspruch, wobei die Säurezahl geringer als 25 ist.

10. Mundpflegezusammensetzung gemäß irgendeinem vorhergehenden Anspruch, wobei die orale Zusammensetzung ein Zahnpflegemittel oder eine Mundspülung ist.

11. Mundpflegezusammensetzung gemäß irgendeinem vorhergehenden Anspruch, worin der Puffer ausgewählt ist aus einer wässrigen Lösung von Bernsteinsäure und dem Natrium- oder Kaliumsalz der Bernsteinsäure, einer wässrigen Lösung der Weinsäure und dem Natrium- oder Kaliumsalz der Weinsäure, einer wässrigen Lösung der Äpfelsäure und dem Natrium- oder Kaliumsalz der Äpfelsäure, einer wässrigen Lösung der Fumarsäure und dem Natrium- oder Kaliumsalz der Fumarsäure, einer wässrigen Lösung der Glutaminsäure und dem Natrium- oder Kaliumsalz der Glutaminsäure, einer wässrigen Lösung der Korksäure und dem Natrium- oder Kaliumsalz der Korksäure, einer wässrigen Lösung der Adipinsäure und dem Natrium- oder Kaliumsalz der Adipinsäure, einer wässrigen Lösung von Sebacinsäure und dem Natrium- oder Kaliumsalz der Sebacinsäure, einer wässrigen Lösung der Glutarsäure und dem Natrium- oder Kaliumsalz der Glutarsäure und einer wässrigen Lösung der Azelainsäure und dem Natrium- oder Kaliumsalz der Azelainsäure, und Kombinationen davon,
wobei der Puffer bei einer Konzentration von 0,05 bis 5,00 Gew.-%, bezogen auf das Gesamtgewicht der Zusammensetzung, vorliegt, und
wobei der pH-Wert der Zusammensetzung von 4,2 bis 4,8 ist.

12. Zusammensetzung gemäß irgendeinem der Ansprüche 1 bis 11 zur Verwendung in einem Verfahren der Bereitstellung von Fluoridionen an die Mundhöhle eines Säugetieres, oder zur Verwendung in einem Verfahren zur Behandlung oder Prävention einer Erkrankung oder Kondition der Mundhöhle eines Säugetieres, oder zur Verwendung in einem Verfahren zur Verminderung der Bildung von dentalen Aushöhlungen in Säugetierzähnen, oder zur Verwendung in einem Verfahren zur Remineralisierung von Säugetierzähnen.

13. Verwendung eines Puffers mit einem pKₐ von weniger als 7,0, wobei der Puffer eine wässrige Lösung einer Säure und eines Salzes der Säure umfasst, und worin das Verhältnis von Säure:Salz zwischen 2:1 und 1:2 ist, zur Steuerung des pH-Wertes einer Mundpflegezusammensetzung, umfassend Aminfluorid, wobei der Puffer den pH-Wert der Zusammensetzung derart kontrolliert, dass dieser größer als 3,5 und geringer als 5,0 ist, und Puffern der Zusammensetzung derart, dass die Zusammensetzung eine Säurezahl größer als 6,0 hat, wobei die Säure ausgewählt ist aus Bernsteinsäure, Milchsäure, Fumarsäure, Glutaminsäure, Korksäure, Adipinsäure, Sebacinsäure, Glutarsäure, Azelainsäure und Kombinationen davon, und wobei die Mundpflegezusammensetzung ein Mundpflegemittel, Zahnpasta, Mundspülung, Mundwäsche, Streifen oder ein festes oder flüssiges Gel ist.

## Revendications

1. Composition de soin buccal comprenant
un véhicule acceptable par voie orale,
un fluorure d'amine, et
un tampon ayant un pKₐ inférieur à 7,0
dans laquelle le tampon comprend une solution aqueuse d'un acide et d'un sel de l'acide, et dans laquelle le rapport acide:sel est compris entre 2:1 et 1:2,
dans laquelle le pH de la composition orale est supérieur à 3,5 et inférieur à 5,0, et
dans laquelle la composition orale a un indice d'acide supérieur à 6,0, et dans laquelle l'acide est choisi parmi l'acide succinique, l'acide tartrique, l'acide malique, l'acide lactique, l'acide fumarique, l'acide glutamique, l'acide subérique, l'acide adipique, l'acide sébacique, l'acide glutarique, l'acide azélaïque et les combinaisons de ceux-ci, et
dans laquelle la composition de soin buccal est un dentifrice, une pâte dentifrice, un rince-bouche, un bain de bouche, une bandelette ou un gel solide ou liquide.

2. Composition de soin buccal selon la revendication 1, dans laquelle l'acide est choisi parmi l'acide fumarique, l'acide succinique, l'acide malique et les combinaisons de ceux-ci.

3. Composition de soin buccal selon l'une quelconque des revendications précédentes, dans laquelle le rapport acide:sel est compris entre 1,5:1 et 1:1,5.

4. Composition de soin buccal selon l'une quelconque des revendications précédentes dans laquelle le tampon est présent à un taux de 0,05 à 5,00 % en poids par rapport au poids total de la composition, éventuellement à un taux de 0,50 à 2,00 % en poids par rapport au poids total de la composition.

5. Composition de soin buccal selon l'une quelconque des revendications précédentes, dans laquelle le fluorure d'amine est l'Olaflur (N'-octadécyltriméthylène-diamine-N,N,N'-tris(2-éthanol)-dihydrofluorure).

6. Composition de soin buccal selon l'une quelconque des revendications précédentes, dans laquelle l'ion fluorure est présent à un niveau de 100 ppm à 10 000 ppm.

7. Composition de soin buccal selon l'une quelconque des revendications précédentes, dans laquelle le pH de la composition orale est supérieur à 4,0, éventuellement dans laquelle le pH de la composition orale est de 4,2 à 4,8.

8. Composition de soin buccal selon l'une quelconque des revendications précédentes, dans laquelle le tampon a un pKₐ de 2,5 à 6,0, éventuellement de 3,5 à 5,6, en outre éventuellement de 4,0 à 5,6, en outre éventuellement encore entre 4,2 et 5,6.

9. Composition de soin buccal selon l'une quelconque des revendications précédentes dans laquelle l'indice d'acide est inférieur à 25.

10. Composition de soin buccal selon l'une quelconque des revendications précédentes, dans laquelle la composition orale est un dentifrice ou un rince-bouche.

11. Composition de soin buccal selon l'une quelconque des revendications précédentes, dans laquelle le tampon est choisi parmi une solution aqueuse d'acide succinique et du sel de sodium ou de potassium de l'acide succinique, une solution aqueuse d'acide tartrique et du sel de sodium ou de potassium de l'acide tartrique, un solution aqueuse d'acide malique et du sel de sodium ou de potassium de l'acide malique, une solution aqueuse d'acide fumarique et du sel de sodium ou de potassium de l'acide fumarique, une solution aqueuse d'acide glutamique et du sel de sodium ou de potassium de l'acide glutamique, une solution aqueuse de l'acide subérique et du sel de sodium ou de potassium de l'acide subérique, une solution aqueuse d'acide adipique et du sel de sodium ou de potassium de l'acide adipique, une solution aqueuse d'acide sébacique et du sel de sodium ou de potassium de l'acide sébacique, une solution aqueuse d'acide glutarique et du sel de sodium ou de potassium de l'acide glutarique, et une solution aqueuse d'acide azélaïque et du sel de sodium ou de potassium de l'acide azélaïque, et les combinaisons de celles-ci,
dans laquelle le tampon est présent à un taux de 0,05 à 5,00 % en poids par rapport au poids total de la composition, et
dans laquelle le pH de la composition est de 4,2 à 4,8.

12. Composition selon l'une quelconque des revendications 1 à 11 pour une utilisation dans un procédé de fourniture d'ions fluorure à la cavité buccale d'un mammifère, ou pour utilisation dans une méthode de traitement ou de prévention d'une maladie ou d'un état de la cavité buccale d'un mammifère, ou pour une utilisation dans un procédé de diminution de la formation de cavités dentaires dans des dents de mammifères, ou pour une utilisation dans un procédé de reminéralisation des dents de mammifères.

13. Utilisation d'un tampon ayant un pKₐ inférieur à 7,0 dans laquelle le tampon comprend une solution aqueuse d'un acide et d'un sel de l'acide, et dans laquelle le rapport acide:sel est compris entre 2:1 et 1:2, pour réguler le pH d'une composition de soin buccal comprenant un fluorure d'amine, le tampon régulant le pH de la composition de sorte que le pH soit supérieur à 3,5 et inférieur à 5,0 et tamponnant la composition de telle sorte que la composition ait un indice d'acide supérieur à 6,0, dans laquelle l'acide est choisi parmi l'acide succinique, l'acide lactique, l'acide fumarique, l'acide glutamique, l'acide subérique, l'acide adipique, l'acide sébacique, l'acide glutarique, l'acide azélaïque et les combinaisons de ceux-ci, et dans laquelle la composition de soin buccal est un dentifrice, une pâte dentifrice, un rince-bouche, un bain de bouche, une bandelette ou un gel solide ou liquide.
